# FASCICULE DE BREVET EUROPEEN

(11) **EP 3 195 021 B1**
(45) Date de publication et mention de la délivrance du brevet: **22.05.2024**
(21) Numéro de dépôt: 14787218.8
(22) Date de dépôt: 12.08.2014
(51) Int. Cl.: G01V 9/00, G01S 13/90, G01C 11/06, G06V 20/13

(54) **PROCÉDÉ DE DÉTECTION DE GISEMENTS D'HYDROCARBURES**
VERFAHREN ZUR ERKENNUNG VON KOHLENWASSERSTOFFLAGERSTÄTTEN
METHOD FOR DETECTING HYDROCARBON DEPOSITS

(43) Date de publication de la demande: 26.07.2017
(73) Titulaire: TotalEnergies OneTech, 92400 Courbevoie (FR)
(72) Inventeur: DHONT, Damien, F-64000 Pau (FR); DUBUCQ, Dominique, F-64000 Pau (FR); GUILLON, Sébastien, F-64000 Pau (FR); PAJOT, Emmanuel, F-64000 Pau (FR); XAVIER, Jean-Paul, F-64000 Pau (FR); MIEGEBIELLE, Véronique, F-64000 Pau (FR)
(74) Mandataire: Plasseraud IP
(86) Numéro de dépôt international: PCT/FR2014/052082
(87) Numéro de publication internationale: WO 2016/024050

(56) Documents cités:
- EP-A2- 1 279 970
- US-A1- 2010 092 241
- US-A1- 2011 213 554
- US-B1- 7 729 561
- N R G Cope ET AL: "OTC-25198-MS SS: Donation of Petrotechnical Data to Universities, Paper: Geohazards And Fluid Seepage Assessment On The Mad Dog Field Using Bathymetry, Ultra-high-resolution Seismic And Satellite Seepage Slick Data", , 8 mai 2014 (2014-05-08), pages 1-28, XP055181527, DOI: http://dx.doi.org/10.4043/25198-MS ISBN: 978-1-61-399308-8 Extrait de l'Internet: URL:https://www.onepetro.org/download/conf erence-paper/OTC-25198-MS?id=conference-pa per/OTC-25198-MS [extrait le 2015-04-08]
- I. R. MACDONALD ET AL: "Natural oil slicks in the Gulf of Mexico visible from space", JOURNAL OF GEOPHYSICAL RESEARCH, vol. 98, no. C9, 15 septembre 1993 (1993-09-15), pages 16351-16364, XP055181574, ISSN: 0148-0227, DOI: 10.1029/93JC01289

## Description

### DOMAINE TECHNIQUE

L'invention se rapporte au domaine de la détection de gisements d'hydrocarbures offshore.

### ARRIÈRE-PLAN TECHNOLOGIQUE

Le besoin croissant en énergie incite à développer de nouveaux moyens de détection et localisation de zones géographiques à fort potentiel en hydrocarbures.

L'une des techniques permettant de détecter de gisements pétroliers sous-marins consiste à surveiller la surface d'une étendue d'eau pour y détecter des traces de fuites naturelles d'hydrocarbures. En effet, un gisement sous-marin peut communiquer avec des fractures jusqu'au fond marin laissant alors s'échapper les hydrocarbures. Ces hydrocarbures ayant une densité inférieure à celle de l'eau, ils remontent en panache dans la colonne d'eau jusqu'à former une fine pellicule de quelques microns d'épaisseur à la surface de l'eau.

La Terre étant recouverte à 75% d'eau, l'observation des traces de fuites d'hydrocarbures en surface repose typiquement sur des images prises depuis des avions ou par des satellites. En la matière, outre l'utilisation de l'imagerie optique, l'imagerie Radar à Synthèse d'Ouverture (RSO) est généralement employée.

Les capteurs radar de moyenne résolution (typiquement 25 m) sont communément utilisés pour détecter des traces de fuites d'hydrocarbures. Ces images couvrent généralement des surfaces de 100 km sur 100 km qui peuvent paraitre réduites pour l'exploration de traces d'huile à l'échelle d'un bassin sédimentaire. Pour détecter les fuites naturelles d'hydrocarbures liquides issus des systèmes pétroliers (qu'on désigne communément par le terme anglais « seep »), il convient alors de les distinguer, d'une part, des autres traces d'huiles liées à des pollutions provoquées par l'homme (dégazage de navires et pollutions de plate-formes qu'on désigne communément par le terme anglais « spill ») et, d'autre part, des sosies (efflorescences algales par exemple) qui peuvent avoir la même signature que les seeps sur les images RSO.

L'utilisation actuelle d'images RSO pour identifier des traces de fuites d'hydrocarbures fournit ainsi des informations avec une fiabilité réduite et qualitatives uniquement, classant des indices d'hydrocarbures détectés sur une échelle de confiance.

COPE, N. R. G. ET AL, "Geohazards And Fluid Seepage Assessment On The Mad Dog Field Using Bathymetry, Ultra-high-resolution Seismic And Satellite Seepage Slick Data", <doi:10.4043/25198-MS> divulgue une évaluation environnementale des géorisques et des phénomènes d'infiltration
de la zone du champ Mad Dog du Golfe du Mexique. Les données disponibles comprennent des interprétations satellitaires des nappes d'infiltration provenant de la grande région de Mad Dog. Une carte annotée des caractéristiques est produite, montrant les emplacements possibles des suintements du fond marin.

Il existe par conséquent un besoin de perfectionnement des techniques de détection s'appuyant sur la surveillance des étendues d'eau afin d'augmenter leur fiabilité et pouvoir mieux détecter des gisements d'hydrocarbures sous-marins.

### EXPOSE DE L'INVENTION

Pour répondre aux problèmes exposés ci-avant, la présente invention propose un procédé de détection et localisation de gisements d'hydrocarbures sous une étendue d' eau, selon la revendication 1.

Le procédé s'appuie ainsi sur un recoupement d'informations issues de différentes images d'une même étendue d'eau prises à des instants différents. L'utilisation d'images prises à des instants différents permet notamment d'éliminer la plupart des traces de fuites d'hydrocarbures transitoires dont l'origine est humaine.

Le procédé repose sur une approche statistique basée sur l'identification des traces de fuites d'hydrocarbures en surface sur chaque image RSO pour générer une carte de détection, puis à combiner les cartes de détections pour produire une carte de localisation des zones géographiques susceptibles de contenir un gisement d'hydrocarbures. En effet, la dérive des hydrocarbures entre leur point d'émission sur le fond marin et leurs traces à la surface de la mer peut fausser la localisation des sources sous-marines de ces hydrocarbures. La dérive des hydrocarbures peut typiquement être sujette à des fluctuations aléatoires liées aux courants marins. En recoupant, par combinaison, les cartes de détection générées à partir de plusieurs images d'une même étendue d'eau, l'invention permet de réduire le biais introduit par cette dérive et d'identifier plus précisément l'emplacement probable de la source sous-marine d'une fuite d'hydrocarbures d'origine naturelle. L'approche statistique augmente ainsi la fiabilité des cartes de localisation de gisements d'hydrocarbures générées.

L'invention permet également de fournir non pas uniquement une information qualitative sur la probabilité de présence d'un gisement d'hydrocarbures, mais également une information quantitative en recoupant par combinaison les images d'une même étendue d'eau. La « combinaison » peut consister en une sommation de probabilités estimées à l'aide des cartes de détection. L'emplacement le plus probable de la source sous-marine révélant un gisement d'hydrocarbures correspond alors aux points où la somme est la plus élevée.

Par ailleurs, la mise en oeuvre du procédé pour analyser des images telles que des images RSO d'une étendue d'eau permet de fournir une information rapide et à bas coût sur la localisation de fuites d'hydrocarbures d'origine naturelle.

Selon un mode de réalisation, les images peuvent couvrir une aire d'au moins 100 kilomètres sur 100 kilomètres.

De telles images dites à large fauchée peuvent être obtenues à moindre coût et couvrent une zone géographique importante. L'utilisation d'images à large fauchée peut également simplifier l'obtention répétée d'images d'une même étendue d'eau. De cette manière, les images fournissent des informations statistiquement plus fiables sur la localisation probable de gisements.

Selon un mode de réalisation, la carte de localisation des sources de fuites d'hydrocarbures peut être produite en combinant des cartes de détection générées à partir d'au moins 50 images de l'étendue d'eau.

En utilisant un nombre d'images d'une même étendue d'eau supérieur à 50, l'approche statistique du procédé objet de l'invention permet d'écarter efficacement la plupart des traces de fuites d'hydrocarbures d'origine humaine. En effet, il a été observé que les nappes d'hydrocarbures en surface s'évaporent en l'espace de quelques heures à quelques jours. Les traces issues du dégazage d'un navire, ou toute autre pollution ponctuelle n'ont alors que peu de chances de se retrouver dans une même zone géographique sur plusieurs images successives prises à des instants différents. Lors de la combinaison des cartes de détection, ces traces d'origine humaine contribuent peu à la somme utilisée pour produire la carte de localisation.

Selon un mode de réalisation, les images peuvent avoir des pixels représentant une aire supérieure à 25 mètres sur 25 mètres.

De telles images sont dites à « moyenne » ou « basse » résolution. En se contentant de telles images, le procédé peut être mis en oeuvre sans avoir recours à la prise d'images spécifiques. Le procédé peut en effet utiliser des banques d'images préexistantes acquises par des satellites d'observation, tels qu'ENVISAT et ERS. La zone géographique couverte peut ainsi être fortement étendue par rapport aux techniques de détection existantes.

Par ailleurs, en utilisant des images ayant une « basse » résolution, une même zone géographique peut être couverte en impliquant un volume de données moins important que des images ayant des pixels représentant des aires plus petites. Cette diminution du volume de données permet de mettre en oeuvre le procédé plus rapidement.

D'autre part, un nombre plus important d'images sur une même étendue d'eau peut alors être pris en compte sans incidence négative perceptible sur la rapidité du procédé.

Selon un mode de réalisation, les images peuvent être prises à des intervalles de temps compris entre un jour et plusieurs mois.

La répétitivité de la prise d'images peut représenter un moyen efficace pour échantillonner la fréquence d'apparition des traces de fuites d'hydrocarbures et distinguer ainsi des indices d'hydrocarbures d'origine naturelle de celles d'origine humaine et de sosies. En obtenant des images réparties dans le temps sur une durée de plusieurs jours ou de plusieurs mois, typiquement 2, 5 ou 10 mois, il est possible de voir si les traces de fuites d'hydrocarbures se répètent dans le temps. Une trace d'origine humaine apparaît typiquement de manière ponctuelle même si elle peut se répéter le long de routes de navigation ou à proximité de plate-formes pétrolières. Le phénomène de fuite d'hydrocarbures issue de gisements naturels est supposé irrégulier dans le temps mais sur de longues durées. Un échantillonnage temporel d'images sur une longue durée permet donc de distinguer des indices d'hydrocarbures naturels, où leur répétitivité doit être significative, des indices d'hydrocarbures d'origine humaine tels que les pollutions de bateaux où la répétitivité doit théoriquement rester faible.

Selon un mode de réalisation, les images peuvent être acquises à l'aide d'un dispositif sensible à des longueurs d'ondes comprises dans le domaine du visible et de l'infrarouge.

Dans le domaine du visible et de l'infrarouge, les traces de fuites d'hydrocarbures apparaissent sous la forme de tâches contrastant avec la couleur ordinaire d'une étendue d'eau. La détection de ces traces dans ce domaine peut s'effectuer quelle que soit la force du vent. Les longueurs d'ondes du domaine visible et de l'infrarouge sont typiquement comprises entre 400 nm et 1 cm.

Selon un autre mode de réalisation, les images peuvent être acquises à l'aide d'un dispositif sensible à des longueurs d'ondes comprises dans le domaine radar.

L'utilisation d'un appareil sensible dans le domaine radar offre un contraste plus important que les dispositifs sensibles à des longueurs d'ondes comprises dans le domaine du visible et de l'infrarouge. Les ondes radar permettent également une observation de nuit et à travers des masses nuageuses. Les longueurs d'ondes du domaine radar sont typiquement comprises entre 1 cm et 1 m.

De manière avantageuse, le procédé peut en outre comprendre, pour chaque image :
- filtrer les traces de fuites d'hydrocarbures identifiées par analyse morphologique pour exclure des traces de fuites d'hydrocarbures d'origine humaine et des sosies.

En écartant les traces de fuites d'hydrocarbures dont l'origine est manifestement humaine, il est possible de rendre plus fiable encore la carte de localisation de fuites d'hydrocarbures générée. Il a été observé que les traces de fuites d'hydrocarbures d'origine humaine sont typiquement rectilignes ou possèdent une courbure régulière C'est notamment le cas de traces ayant pour origine une pollution par des navires. Les traces dessinent un motif plus complexe en surface lorsqu'elles ont une origine naturelle du fait des courants marins à l'origine de la dérive des hydrocarbures remontant à la surface. Il est également possible d'écarter les traces de fuites d'hydrocarbures correspondant à des sosies, c'est-à-dire des traces en surface qui n'ont que l'apparence d'une trace de fuites d'hydrocarbures, telles que par exemple des efflorescence algales.

Selon un mode de réalisation, l'identification de traces de fuites d'hydrocarbures dans une image peut comprendre la recherche des traces à des endroits où souffle un vent de vitesse généralement comprise entre 3 mètres par seconde et 10 mètres par seconde.

L'identification de traces de fuites d'hydrocarbures sur des images RSO peut s'avérer peu fiable en l'absence de tout vent ou lorsque le vent dépasse une valeur d'environ 10 mètres par seconde. En effet, en l'absence de tout vent, la surface d'une étendue d'eau ne comprend généralement pas de clapot. Ainsi, la nappe d'hydrocarbures et la surface de l'eau environnante constituent toutes les deux des surfaces lisses qui réfléchissent des ondes radar comme un miroir plan. Il est alors difficile de distinguer la trace de la fuite d'hydrocarbures de l'eau sur une image RSO quand le vent est absent. En présence d'un vent fort ayant une vitesse supérieure à 10 mètres par seconde, la surface d'une étendue d'eau est soumise à un clapot important. De même, le clapot affecte la nappe d'hydrocarbures à la surface de l'eau. La réflexion de l'onde radar est alors diffuse pour ces deux entités qui ne peuvent alors pas facilement être distinguées sur l'image RSO. Par contre, en présence de vents ayant une vitesse intermédiaire comprise entre 3 mètres par seconde et 10 mètres par seconde, l'eau de mer est soumise à du clapot alors que la nappe d'hydrocarbures conserve une surface lisse réfléchissant les ondes radar comme un miroir plan. Des traces de fuites d'hydrocarbures sont alors facilement identifiables du fait d'un fort contraste par rapport au reste de l'eau sur une image RSO.

Selon un mode de réalisation, des images de plusieurs zones géographiques peuvent être acquises pour générer des cartes de détection respectives, la combinaison des cartes de détection générées pour l'une des zones géographiques comprenant une normalisation en fonction du nombre d'images acquises pour ladite zone géographique.

Une telle étape de normalisation permet de renforcer la fiabilité des cartes de localisation. Plus le nombre d'images d'une étendue d'eau est élevé, plus les données cumulées contenues dans ces images offrent des informations statistiquement fiables. Il peut donc être avantageux d'attribuer un coefficient de fiabilité plus important aux cartes générées à partir d'un nombre élevé d'images. Une étendue d'eau pour laquelle seul un nombre faible d'images est disponible, typiquement inférieur à 10 images, peut fournir des informations trompeuses car peu fiables statistiquement. La normalisation permet de corriger de tels biais et n'attribuer des probabilités de présence élevées qu'aux zones géographiques couvertes à l'aide d'un nombre suffisant d'images.

Selon l'invention, le traitement de chaque image sur la base au moins d'un critère d'éloignement aux traces de fuites d'hydrocarbures identifiées comprend l'affectation à chaque point i de l'image d'une valeur calculée en fonction de la distance du point i à la trace de la fuite d'hydrocarbures identifiée la plus proche du point i.

L'éloignement d'un point à une trace de fuite d'hydrocarbures est un moyen simple pour définir une probabilité de présence d'une source d'hydrocarbures. Plus particulièrement, en mesurant la distance d'un point i à la trace de la fuite d'hydrocarbures la plus proche, une carte de distances peut être générée. Ces distances peuvent être associées à une fonction, par exemple une fonction décroissante à mesure que la position du point i est éloignée d'une trace, pour définir des probabilités de présence d'un gisement sur la carte de détection.

Plus particulièrement, la valeur affectée à un point i de l'image peut être proportionnelle à exp[-dᵢ²/(2σ²)], où dᵢ désigne la distance du point i à la trace de la fuite d'hydrocarbures la plus proche, et σ² représente une variance représentative d'une zone d'influence de la trace de la fuite d'hydrocarbures.

Le paramètre ajustable σ de la fonction probabilité définissant la valeur affectée à un point i de l'image permet de prendre en compte l'influence de courants marins et d'autres paramètres environnementaux susceptibles d'influencer la dérive d'une fuite d'hydrocarbures. Par exemple, le paramètre σ² peut être fixé égal à 7,5 kilomètres, ce qui permet de prendre en compte efficacement la plupart des dérives liées au courant tout en considérant des aires peu étendues pour localiser des gisements d'hydrocarbures.

Selon un mode spécifique de réalisation, la combinaison des cartes de détection générées peut comprendre, pour chaque point i, la sommation des valeurs affectées au point i sur chaque carte de détection.

Cette sommation des valeurs affectées à chaque point sur les cartes de détection produit de nouvelles valeurs représentatives de la répétitivité d'une trace dans le temps. La somme obtenue a donc une valeur basse lorsque les traces n'apparaissent que ponctuellement et une valeur haute lorsque les traces apparaissent régulièrement. Cette dépendance avec la répétitivité élimine naturellement la contribution à la probabilité de présence des « spills » et augmente la visibilité des emplacements de gisements potentiels.

Par ailleurs, lorsque la position des « seeps » subit des fluctuations dans le temps, la sommation permet de réduire l'influence sur la probabilité de présence affichée sur une carte de localisation des traces fortement déviées du gisement potentiel. De cette manière, la sommation des valeurs affectées à chaque point fournit une information de localisation améliorée de gisements potentiels.

### DESCRIPTIF DES FIGURES

Le procédé objet de l'invention sera mieux compris à la lecture de la description qui suit d'exemples de réalisations présentés à titre illustratif, aucunement limitatifs, et à l'observation des dessins ci-après sur lesquels :
- la figure 1 est une représentation en perspective schématique en coupe d'une étendue d'eau, incluant un gisement sous-marin présentant une fuite d'hydrocarbures laissant en surface de l'étendue d'eau une trace de fuite d'hydrocarbures ; et
- la figure 2 est une représentation schématique en vue de dessus de différentes traces d'hydrocarbures d'origine naturelle et de pollutions sur une étendue d'eau ; et
- la figure 3a est une représentation schématique en vue de côté d'un plan d'eau comprenant des fuites d'hydrocarbures s'échappant par une faille d'un gisement sous-marin, et d'une nappe d'hydrocarbures en surface ; et
- les figures 3b et 3c sont des représentations schématiques en vue de côté d'une nappe d'hydrocarbures en surface d'une étendue d'eau soumise à différentes vitesses de vent ; et
- la figure 4 est une représentation schématique : A d'une carte de distances des points d'une image aux traces de fuites d'hydrocarbures de cette image, et B d'une carte de détection des fuites d'hydrocarbures obtenue par application d'une fonction probabilité à la carte de distances A ; et
- les figures 5a et 5b sont des représentations schématiques de cartes de localisation de fuites d'hydrocarbures respectivement avant normalisation et après normalisation.

Pour des raisons de clarté, les dimensions des différents éléments représentés sur ces figures ne sont pas nécessairement en proportion avec leurs dimensions réelles. Sur les figures, des références identiques correspondent à des éléments identiques.

### DESCRIPTION DÉTAILLÉE

L'invention se rapporte à un procédé d'analyse d'images de la surface d'une étendue d'eau permettant de détecter des traces de fuites d'hydrocarbures d'origine naturelle. Ce procédé permet également de localiser la position la plus probable de la source de ces fuites d'hydrocarbures. L'invention s'appuie sur une approche statistique permettant de discriminer de manière automatique, rapidement et à faible coût les traces de fuites d'hydrocarbures d'origine naturelle (« seeps ») de celles d'origine humaine (« spills »).

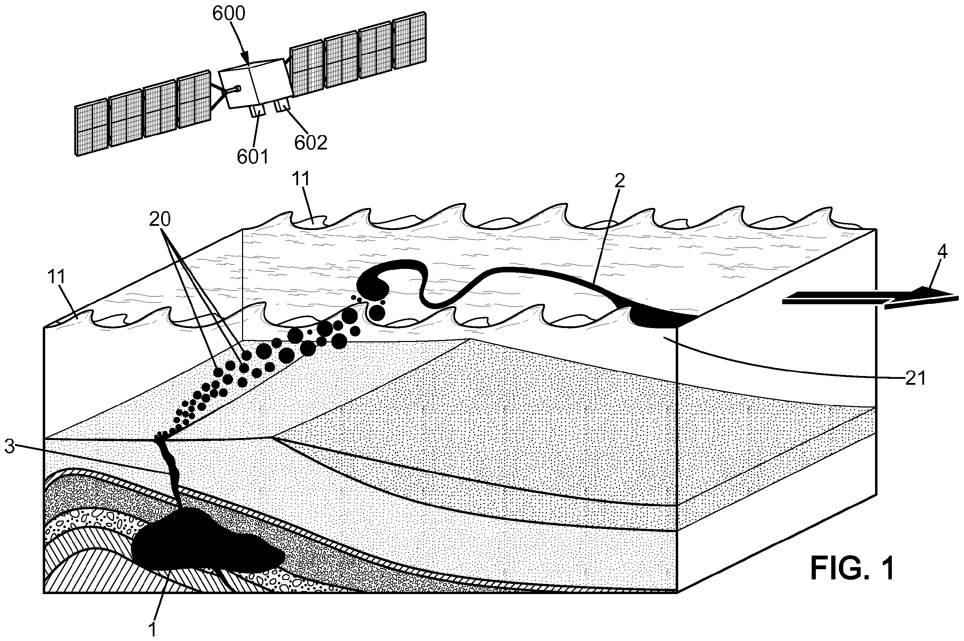

Des gisements sous-marins d'hydrocarbures 1 peuvent généralement rejeter une faible quantité d'hydrocarbures à travers des fractures 3 dans le fond marin. La figure 1 illustre schématiquement sur une coupe vue en perspective une fuite d'hydrocarbures 20 à travers une telle fracture 3 à partir d'un gisement 1. La densité des hydrocarbures, tels que le pétrole, est souvent inférieure à celle de l'eau, et en particulier de l'eau de mer. Pour cette raison, les hydrocarbures 20 s'élèvent depuis la fracture 3 vers la surface 11, pour y former des traces 2 de fuites d'hydrocarbures.

Ces traces 2 de fuites d'hydrocarbures présentent généralement un profil ondulé avec des méandres, du fait de la dérive aléatoire, au gré des courants marins 4, des hydrocarbures 20 remontant vers la surface 11.

En surface, les traces 2 de fuites d'hydrocarbures forment des nappes 21 qui restent lisses tant que le vent n'excède pas une vitesse de l'ordre de 10 mètres par seconde.

Le procédé objet de l'invention utilise une banque d'images de la surface d'une étendue d'eau afin d'y identifier des « seeps ». Ce procédé détecte ensuite les fuites d'hydrocarbures d'origine naturelle et localise leur point d'émission le plus probable.

L'obtention des images d'une étendue d'eau peut se faire au moyen de survols par avion, ou bien en accédant aux images prises par des satellites d'observation 600, tels que ENVISAT ou ERS. Ces appareils sont ou peuvent être équipés de capteurs optiques 601 et/ou de capteurs radars 602.

Le choix de la taille des images, leur résolution et leur nombre pour une même étendue d'eau sont des paramètres ajustables de l'invention.

Les images radar à synthèse d'ouverture (RSO) couramment utilisées pour identifier des traces de fuites d'hydrocarbures ont une résolution dite moyenne. Une résolution moyenne correspond ici à des pixels sur l'image représentant une aire d'environ 25 m sur 25 m, c'est-à-dire une capacité à détecter des objets ayant une taille de plus de 25 m. Ces capteurs peuvent couvrir avec cette résolution une aire de 100 km sur 100 km.

Le procédé objet la présente invention peut fonctionner avec des images ayant des résolutions nominales basses, c'est-à-dire ayant des pixels représentant une aire de 150 m sur 150 m, et couvrant sur une image une aire de 400 km sur 400 km. Ainsi, la zone géographique étudiée en est élargie et la quantité d'informations à traiter par image réduite. Il a en effet été constaté que les seeps 2 s'étendent généralement sur plusieurs kilomètres de long et plusieurs centaines de mètres de large, ce qui les rend détectables sur des images à basse résolution telles que décrites ci-avant.

Par ailleurs, le nombre d'images disponibles avec cette résolution est grand et elles couvrent une superficie plus importante. Les satellites d'observation ont en effet accumulé de telles images horodatées avec plus de 50 images pour une même zone géographique sur une partie des mers et océans au voisinage des côtes. L'invention peut donc être mise en oeuvre sans avoir recours à la mise en place de moyens d'observation coûteux pour constituer des banques d'images.

Il est avantageux de disposer d'un nombre élevé, de préférence supérieur à 50, d'images d'une même étendue d'eau prises à des instants différents. Les « seeps » 2 se reproduisent de manière aléatoire et répétée. Les « spills » 201, 202 surviennent de manière ponctuelle du fait par exemple d'une pollution créée par l'Homme.

Ainsi, en disposant d'un nombre supérieur à 50 d'images prises à des instants différents d'une même étendue d'eau, il est possible de distinguer les « seeps » et les « spills », à l'aide de la fréquence d'occurrence des traces de fuites d'hydrocarbures.

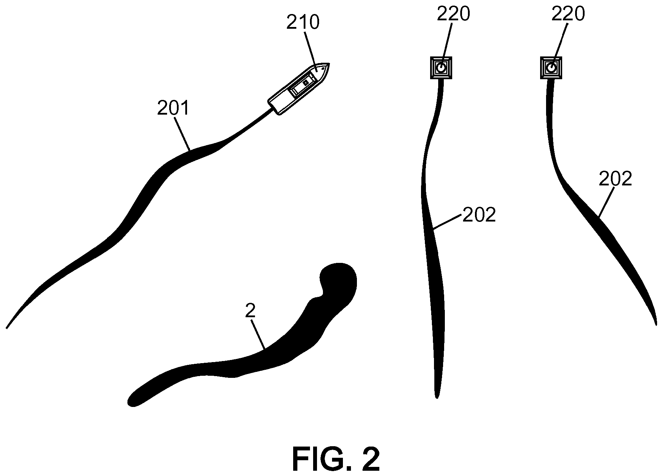

La figure 2 illustre schématiquement plusieurs exemples de traces 2, 201, 202 de fuites d'hydrocarbures pouvant typiquement apparaitre sur une image. Seule la trace 2 a une origine naturelle. Elle se distingue des autres par ses ondulations et sa forme peu régulière. La trace 201 est rectiligne et suit le sillage d'un navire 210, qui peut être identifiable sur une image. En l'absence de navire sur l'image, la présence d'une trace de forme similaire à la trace 201 peut être attribuée à une origine humaine si elle se situe sur une voie de navigation connue. Les traces 202 sont également longilignes, rectilignes ou courbées, et sont liées à une pollution depuis une plate-forme 220. Les plates-formes 202, éléments fixes, peuvent être identifiables sur les images.

Une étape optionnelle de discrimination préalable des traces 201, 202 de fuites d'hydrocarbures dont l'origine est manifestement artificielle peut être mise en oeuvre. Cette sélection peut s'opérer sur des critères de forme ou bien en croisant les traces 201, 202 avec la position de plates-formes ou voies de navigation connues. Ce filtrage par analyse morphologique réduit la quantité de données à prendre en compte lors de la mise en oeuvre des étapes ultérieures du procédé.

Cependant, certaines traces peuvent avoir l'apparence d'un « seep » sans être issues d'un gisement d'hydrocarbures. Ceci peut, par exemple, survenir en présence de forts courants de surface déformant les traces laissées par les navires ou en présence de cellules de pluie, de fronts océaniques ou d'efflorescences algales. Pour distinguer ces traces des « seeps », l'invention exploite la répétitivité des données RSO acquises pour une même étendue d'eau.

Il est alors avantageux de disposer d'images RSO d'une même étendue d'eau prises sur une durée longue s'étalant sur plusieurs mois voire plusieurs années. Une telle durée d'observation permet de réduire les biais de localisation liés à des conditions climatiques particulières ou des effets saisonniers sur les courants marins à l'origine de dérives. Il est aussi avantageux de disposer d'images prises à des intervalles de temps peu espacés. Des intervalles de temps s'échelonnant sur des durées comprises entre un jour et quelques mois pour la prise d'images permettent d'atteindre un bon rapport fréquence d'acquisition/durée d'acquisition.

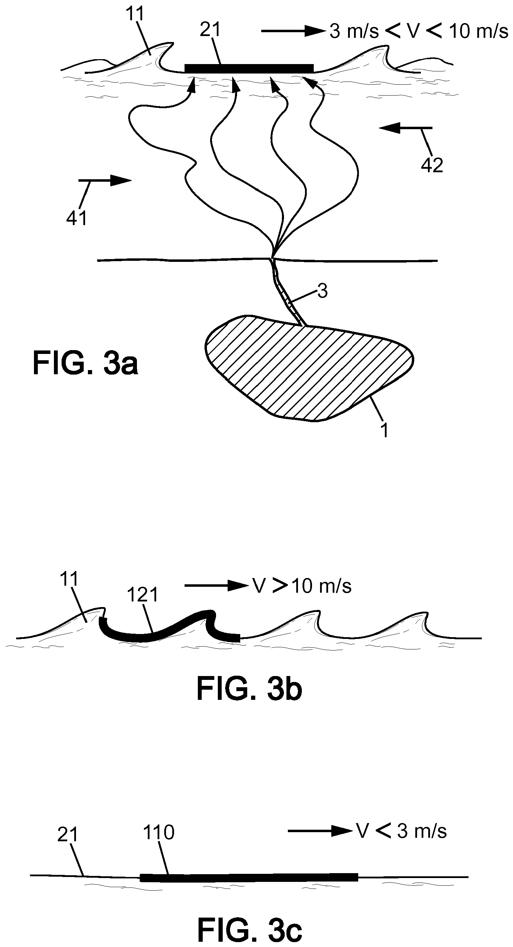

Le procédé objet de l'invention identifie sur chaque image acquise les traces 2 de fuites d'hydrocarbures. L'identification s'effectue soit sur des images acquises avec un capteur optique 601 sensible à des longueurs d'ondes comprises dans le domaine regroupant le domaine visible et infrarouge, soit sur des images acquises avec un capteur radar 602. Les images acquises avec un capteur « optique » permettent d'identifier des nappes 21 d'hydrocarbures quelle que soit la vitesse du vent, mais de jour et par temps dégagé. Les images acquises avec un capteur « radar » permettent de voir des nappes 21 d'hydrocarbures de jour comme de nuit, et à travers des masses nuageuses, mais en présence de conditions de vent particulières, comme représenté schématiquement sur les figures 3a à 3c.

La figure 3a représente une étendue d'eau balayée en surface par un vent ayant une vitesse comprise entre 3 mètres par seconde et 10 mètres par seconde. Dans une telle configuration, la nappe 21 d'hydrocarbures reste lisse tandis que l'eau environnante est perturbée par du clapot en surface. Sur une image radar, cette situation permet de voir avec un fort contraste la nappe 21 qui réfléchit les ondes comme un miroir. La surface déformée de l'eau environnante n'agissant pas comme un miroir elle est perçue avec une teinte différente sur l'image radar.

La figure 3b représente une étendue d'eau balayée en surface par un vent ayant une vitesse supérieure à 10 mètres par seconde. La nappe 121 d'hydrocarbures affectée par le clapot possède une surface rugueuse semblable à celle de la surface 11 de l'eau environnante. La nappe 121 et la surface 11 de l'eau auront alors typiquement la même teinte sur l'image radar.

La figure 3b représente une étendue d'eau balayée en surface par un vent ayant une vitesse inférieure à 3 mètres par seconde. La nappe 21 reste lisse tout comme la surface 110 de l'eau environnante. Dans cette situation, l'image radar représente la nappe 21 et la surface 110 de l'eau avec la même teinte, et il n'est pas possible de distinguer ces deux entités sur une image radar. Cependant, une image optique permettrait de percevoir dans une couleur différente une nappe 21 même en présence d'un tel vent faible.

Il apparaît donc que les meilleures conditions d'observation se présentent lorsque le vent balayant une étendue d'eau a une vitesse comprise entre 3 mètres par seconde et 10 mètres par seconde. Cette configuration est indifféremment adaptée pour une imagerie optique ou radar. Par conséquent, les images acquises peuvent également indiquer le champ du vent balayant la zone géographique représentée sur une image. Dans la pratique, ce champ de vent peut être extrait d'une image à l'aide d'outils connus.

Après avoir identifié les traces 2 de fuites d'hydrocarbures sur les images acquises, le procédé propose, pour chaque image, de générer une carte de détection mettant en évidence, autour de chaque trace 2 de fuites d'hydrocarbures, l'emplacement probable de la source sous-marine des fuites d'hydrocarbures.

Comme représenté sur les figures 1 et 3a, les hydrocarbures 20 s'échappant par une fracture 3 d'un gisement 1 remontent à la surface en étant soumis aux courants marins 4, 41, 42. Ces courants peuvent être à l'origine d'une dérive importante des traces 2 de fuites d'hydrocarbures, qui ne se retrouvent alors pas à l'aplomb des gisements 1 leur donnant naissance.

Le procédé génère une carte de détection de l'emplacement probable de la source de chaque trace 2 de fuites d'hydrocarbures au moins sur la base d'un critère d'éloignement d'un point de la surface aux traces 2 identifiées.

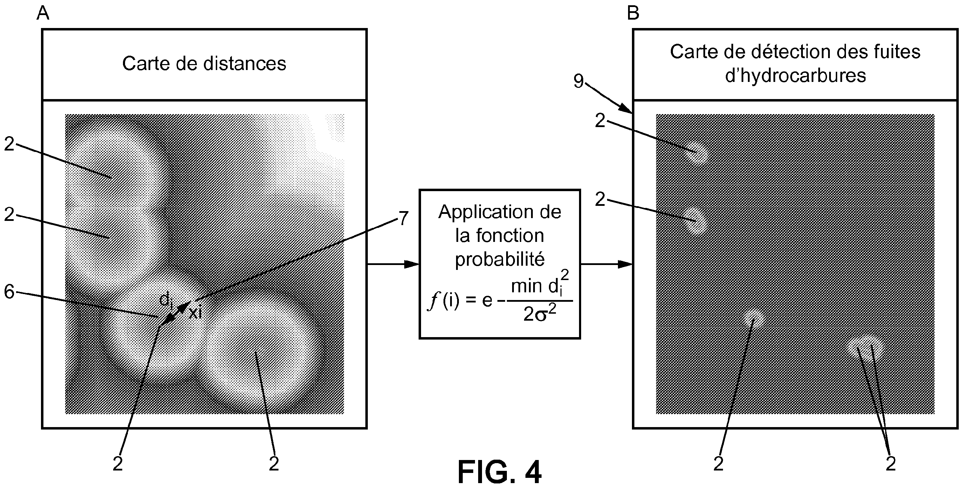

Un mode de réalisation représenté sur la figure 4 A consiste à générer une carte de distances dans laquelle chaque point 7, i d'une image est indiqué par une couleur qui est fonction de son éloignement 6, dᵢ à la trace 2 de fuites d'hydrocarbures la plus proche.

En fonction des connaissances disponibles sur la zone géographique imagée, il est ensuite possible de générer une carte de détection 9 telle que celle représentée sur la figure 4 B en appliquant à chaque point i de l'image de la figure 4 A une fonction probabilité.

À titre d'exemple, une fonction Gaussienne telle que la fonction f définie pour tout point i par l'expression f(i)=exp[-dᵢ²/(2σ²)] peut être utilisée. Dans cette expression, dᵢ désigne la distance 6 du point 7, i à la trace 2 la plus proche de ce point, et σ² représente une variance, définissant une zone d'influence de la trace de la fuite d'hydrocarbures. Une zone d'influence typique peut être égale à 8 km, la valeur de σ² pouvant, dans cet exemple, être fixée à 7,5 km. Le paramètre σ est une variable d'ajustement qui peut être modifiée si les conditions de la zone géographique représentée sur l'image le suggèrent. Par ailleurs, dans l'éventualité où un courant de vecteur connu serait présent, il est possible d'appliquer toute autre fonction probabilité prenant en compte ces données supplémentaires.

Les cartes de détection 9 ainsi obtenues constituent un ensemble de données utilisables pour un recoupement permettant par combinaison de converger vers une position plus probable et fiable du point-source d'où provient la trace 2 de fuites d'hydrocarbures. La « combinaison » peut consister en une sommation des probabilités affichées sur les cartes de détection 9.

À titre d'exemple, la sommation des probabilités peut être effectuée point par point à partir de chaque carte de détection générée pour une même étendue d'eau.

L'étape de combinaison permet de générer une grille de probabilités d'occurrence d'une trace d'hydrocarbures correspondant à la répétitivité du phénomène de fuite au cours du temps. Pour chaque point 7, i la probabilité de présence d'un gisement sur chaque carte de détection est prise en compte en vue d'obtenir par sommation une valeur intégrant les variations de cette probabilité au cours du temps. De cette manière, les traces 2 de fuites d'hydrocarbures d'origine humaine contribuent moins à la probabilité de présence d'un gisement que les traces 2 de fuites d'hydrocarbures d'origine naturelle, qui se retrouvent plus souvent sur les images successives comme évoqué ci-avant.

La carte de localisation générée à partir de cette combinaison s'apparente à une image de superposition, aussi appelée image de « stack » selon la terminologie anglo-saxonne.

L'approche statistique du procédé de l'invention implique l'utilisation d'un nombre élevé, de préférence supérieur à 50, d'images d'une même étendue d'eau. Pour différencier les cartes de localisation en fonction du nombre d'images de détection disponibles pour les générer, une étape de normalisation peut être mise en oeuvre.

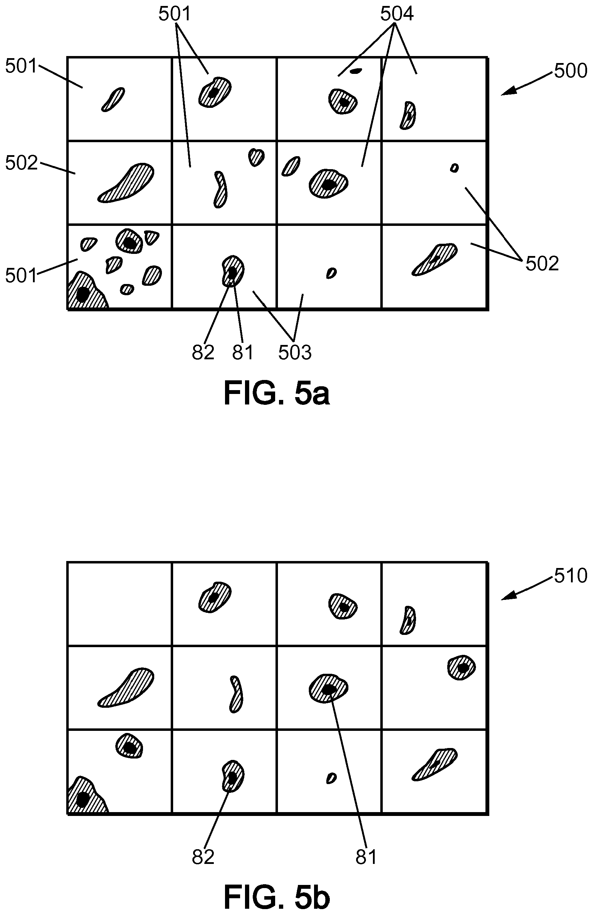

Les figures 5a et 5b représentent respectivement une carte de localisation obtenue avant normalisation et après normalisation.

La figure 5a représente une carte de localisation 500 comprenant douze zones géographiques différentes. Dans chaque zone, des formes pleines 81 ou hachurées 82 représentent des emplacements probables de gisements d'hydrocarbures. Les formes pleines 81 sont celles pour lesquelles la probabilité de présence d'un gisement a été calculée comme étant la plus élevée.

Chaque zone géographique a été cartographiée à l'aide d'un nombre différent d'images. Les zones géographiques 501 ont été obtenues avec moins de dix images, les zones géographiques 502, avec plus de dix mais moins de trente images, les zones géographiques 503 avec plus de trente mais moins de cinquante images et les zones géographiques 504 avec plus de cinquante images. La carte de localisation de la figure 5a indique en apparence que les zones géographiques 501 comprennent des sites possédant une forte probabilité de présence de gisements d'hydrocarbures.

La figure 5b représente une carte de localisation 510 obtenue après normalisation de la carte de localisation 500. La normalisation consiste à corriger les valeurs affectées aux points d'une carte de localisation représentant une zone géographique par un coefficient fonction du nombre d'images utilisées pour générer la carte de localisation dans cette zone géographique.

Il apparaît sur la figure 5b que les sites considérés comme potentiellement riches en hydrocarbures dans les zones géographiques 501 d'après la figure 5a s'avèrent être des sites moins prometteurs pour un gisement d'hydrocarbures après normalisation. La normalisation permet ainsi de corriger des biais liés à un nombre insuffisant de données.

L'invention décrite ci-avant permet d'obtenir une carte des emplacements probables de gisements d'hydrocarbures en fournissant une information quantitative et non seulement qualitative. Le procédé mis en oeuvre ne nécessite pas l'utilisation de ressources coûteuses et peut être mise en oeuvre rapidement. L'approche statistique du procédé permet de réduire sur les cartes de localisation la contribution aux valeurs de probabilité de présence de gisements d'hydrocarbures des traces de fuites d'hydrocarbures d'origine humaine.

Lorsqu'une carte de localisation a été obtenue à partir de données RSO à basse résolution, il est possible de compléter l'analyse d'images d'une étendue d'eau en utilisant des images offrant une meilleure résolution, afin de réduire l'étendue de l'aire considérée pour l'emplacement probable d'un gisement. Par ailleurs, une fois qu'une carte de localisation fiable a été générée, il est possible de superposer cette carte avec une carte correspondante du fond de l'étendue d'eau, afin d'identifier plus précisément l'origine probable de la fuite d'hydrocarbures à partir de la bathymétrie du milieu.

L'invention ne se limite pas aux modes de réalisation décrits ci-avant. Notamment, le procédé de l'invention peut être mis en oeuvre avec des images possédant de meilleures résolutions et/ou couvrant des aires de plus faible étendue.

## Revendications

1. Procédé de détection et localisation de gisements (1) d'hydrocarbures sous une étendue d'eau, le procédé comprenant :
- acquérir des images superposables d'une surface de l'étendue d'eau prises à des instants différents ;
- pour chaque image :
• identifier des traces (2, 201, 202) de fuites d'hydrocarbures ;
• générer une carte de détection (9) indicative de probabilités de présence d'une source d'hydrocarbures (20) autour des traces (2) de fuites d'hydrocarbures identifiées, par traitement de l'image au moins sur la base d'un critère d'éloignement aux traces (2) de fuites d'hydrocarbures identifiées
- combiner les cartes de détection générées pour produire une carte de localisation (500, 510) des sources de fuites d'hydrocarbures,
le procédé **caractérisé en ce que:**
le traitement de chaque image sur la base au moins d'un critère d'éloignement aux traces (2) de fuites d'hydrocarbures identifiées comprend l'affectation à chaque point i de l'image d'une valeur calculée en fonction de la distance dᵢ du point i à la trace de la fuite d'hydrocarbures identifiée la plus proche du point i.

2. Procédé selon la revendication 1, dans lequel les images couvrent une aire d'au moins 100 kilomètres sur 100 km.

3. Procédé selon l'une quelconque des revendications précédentes, dans lequel la carte de localisation (500, 510) des sources de fuites d'hydrocarbures est produite en combinant des cartes de détection générées à partir d'au moins 50 images de l'étendue d'eau.

4. Procédé selon l'une quelconque des revendications précédentes, dans lequel les images ont des pixels représentant une aire supérieure à 25 mètres sur 25 mètres.

5. Procédé selon l'une quelconque des revendications précédentes, dans lequel les images sont prises à des intervalles de temps compris entre un jour et plusieurs mois.

6. Procédé selon l'une quelconque des revendications précédentes, dans lequel les images sont acquises à l'aide d'un dispositif (601) sensible à des longueurs d'ondes comprises dans le domaine du visible et de l'infrarouge.

7. Procédé selon l'une quelconque des revendications précédentes, dans lequel les images sont acquises à l'aide d'un dispositif (602) sensible à des longueurs d'ondes comprises dans le domaine radar.

8. Procédé selon l'une quelconque des revendications précédentes, comprenant en outre, pour chaque image :
• filtrer les traces de fuites d'hydrocarbures identifiées par analyse morphologique pour exclure des traces de fuites d'hydrocarbures d'origine humaine et des sosies.

9. Procédé selon l'une quelconque des revendications précédentes, dans lequel l'identification de traces (2) de fuites d'hydrocarbures dans une image comprend la recherche des traces à des endroits où souffle un vent de vitesse comprise entre 3 mètres par seconde et 10 mètres par seconde.

10. Procédé selon l'une quelconque des revendications précédentes, dans lequel des images de plusieurs zones géographiques sont acquises pour générer des cartes de détection respectives, la combinaison des cartes de détection générées pour l'une des zones géographiques comprenant une normalisation en fonction du nombre d'images acquises pour ladite zone géographique.

11. Procédé selon l'une des revendication précédentes, dans lequel la valeur affectée à un point i de l'image est proportionnelle à exp[-dᵢ²/(2σ²)], où dᵢ désigne la distance du point i à la trace (2) de la fuite d'hydrocarbures la plus proche, et σ² représente une variance représentative d'une zone d'influence de la trace de la fuite d'hydrocarbures.

12. Procédé selon l'une des revendications précédentes, dans lequel la combinaison des cartes de détection (9) générées comprend, pour chaque point i, la sommation des valeurs affectées au point i sur chaque carte de détection.

## Patentansprüche

1. Verfahren zur Detektion und Lokalisation von Kohlenwasserstoff-Lagerstätten (1) unter einer Wasserausdehnung, wobei das Verfahren aufweist:
- Erfassen von übereinander positionierbaren Bildern einer Oberfläche der Wasserausdehnung, die zu unterschiedlichen Zeitpunkten aufgenommen werden;
- für jedes Bild:
o Identifizieren von Kohlenwasserstoff-Austrittsspuren (2, 201, 202);
o Generieren einer Detektionskarte (9), die Wahrscheinlichkeiten für ein Vorhandensein einer Kohlenwasserstoff-Quelle (20) in der Umgebung der identifizierten Kohlenwasserstoff-Austrittsspuren (2) anzeigt, durch Verarbeiten des Bilds zumindest auf Basis eines Kriteriums eines Abstands zu den identifizierten Kohlenwasserstoff-Austrittsspuren (2);
- Kombinieren der generierten Detektionskarten, um eine Lokalisationskarte (500, 510) für Kohlenwasserstoff-Austrittsquellen zu erstellen,
wobei das Verfahren **dadurch gekennzeichnet ist, dass**:
das Verarbeiten jedes Bilds auf Basis zumindest eines Kriteriums eines Abstands zu den identifizierten Kohlenwasserstoff-Austrittsspuren aufweist: Zuweisen eines Werts zu jedem Punkt i des Bilds, wobei der Wert berechnet wird als Funktion des Abstands dᵢ des Punkts i zu derjenigen Kohlenwasserstoff-Austrittsspur, die als die dem Punkt i am nächsten liegende identifiziert worden ist.

2. Verfahren nach Anspruch 1, in welchem die Bilder ein Gebiet von mindestens 100 Kilometer auf 100 km abdecken.

3. Verfahren nach einem der vorstehenden Ansprüche, in welchem die Kohlenwasserstoff-Austrittsquellen-Lokalisationskarte (500, 510) durch Kombinieren von Detektionskarten, die ausgehend von mindestens 50 Bildern der Wasserausdehnung generiert worden sind, erstellt wird.

4. Verfahren nach einem der vorstehenden Ansprüche, in welchem die Bilder Pixel haben, die ein Gebiet von mehr als 25 Meter auf 25 Meter repräsentieren.

5. Verfahren nach einem der vorstehenden Ansprüche, in welchem die Bilder in Zeitintervallen zwischen einem Tag und mehreren Monaten aufgenommen werden.

6. Verfahren nach einem der vorstehenden Ansprüche, in welchem die Bilder mithilfe einer Vorrichtung (601) erfasst werden, die für Wellenlängen, die im sichtbaren Bereich und im Infrarot liegen, empfindlich ist.

7. Verfahren nach einem der vorstehenden Ansprüche, in welchem die Bilder mithilfe einer Vorrichtung (602) erfasst werden, die für Wellenlängen, die im Radarbereich liegen, empfindlich ist.

8. Verfahren nach einem der vorstehenden Ansprüche, aufweisend ferner für jedes Bild:
∘ Filtern der identifizierten Kohlenwasserstoff-Austrittsspuren, um Kohlenwasserstoff-Austrittsspuren menschlichen Ursprungs und von Doppelgängern auszuschließen.

9. Verfahren nach einem der vorstehenden Ansprüche, in welchem das Identifizieren von Kohlenwasserstoff-Austrittsspuren (2) in einem Bild aufweist: Suchen von Spuren an Orten, wo ein Wind mit einer Geschwindigkeit zwischen 3 Meter pro Sekunde und 10 Meter pro Sekunde weht.

10. Verfahren nach einem der vorstehenden Ansprüche, in welchem Bilder von mehreren geographischen Zonen erfasst werden, um jeweilige Detektionskarten zu generieren, wobei das Kombinieren der Karten, die für eine der geographischen Zone generiert worden sind, eine Normalisierung als Funktion der Anzahl der für diese geographische Zone erfassten Bilder aufweist.

11. Verfahren nach einem der vorstehenden Ansprüche, in welchem der Wert, der einem Punkt i des Bilds zugewiesen wird, proportional zu exp[-dᵢ²/(2σ²)] ist, wobei dᵢ den Abstand des Punkts i zu der am nächsten liegenden Kohlenwasserstoff-Austrittsspur (2) bezeichnet und σ² eine Varianz, die für eine Einflusszone der Kohlenwas serstoff-Austritts spur repräsentativ ist, repräsentiert.

12. Verfahren nach einem der vorstehenden Ansprüche, in welchem das Kombinieren der generierten Detektionskarten (9) für jeden Punkt i die Summierung der Werte, die dem Punkt i auf jeder Detektionskarte zugewiesen sind, aufweist.

## Claims

1. Method for detecting and locating hydrocarbon deposits (1) under a body of water, the method comprising:
- acquiring superposable images of a surface of the body of water taken at different times;
- for each image:
• identifying traces (2, 201, 202) of hydrocarbon leaks;
• generating a detection map (9) indicative of probabilities of the presence of a hydrocarbon source (20) around the identified traces (2) of hydrocarbon leaks, by processing the image at least on the basis of a criterion of distance from the identified traces (2) of hydrocarbon leaks;
- combining the detection maps generated to produce a location map (500, 510) of the sources of hydrocarbon leaks,
the method being **characterised in that**
the processing of each image on the basis of at least one criterion of distance from the traces (2) of identified hydrocarbon leaks comprises assigning to each point i of the image a value calculated on the basis of the distance dᵢ of the point i from the trace of the identified hydrocarbon leak closest to point i.

2. Method according to claim 1, wherein the images cover an area of at least 100 km by 100 km.

3. Method according to any one of the preceding claims, wherein the hydrocarbon leak source location map (500, 510) is produced by combining detection maps generated from at least 50 images of the body of water.

4. Method according to any one of the preceding claims, wherein the images have pixels representing an area greater than 25 metres by 25 metres.

5. Method according to any one of the preceding claims, wherein the images are taken at time intervals of between one day and several months.

6. Method according to any one of the preceding claims, wherein the images are acquired using a device (601) sensitive to wavelengths included in the visible and infrared range.

7. Method according to any one of the preceding claims, wherein the images are acquired using a device (602) sensitive to wavelengths included in the radar range.

8. Method according to any one of the preceding claims, further comprising for each image:
• filtering traces of hydrocarbon leaks identified by morphological analysis to exclude traces of hydrocarbon leaks of human origin and look-alike images.

9. Method according to any one of the preceding claims, wherein identifying traces (2) of hydrocarbon leaks in an image comprises searching for traces at locations where a wind at a speed of between 3 metres per second and 10 metres per second is blowing.

10. Method according to any one of the preceding claims, wherein images of a plurality of geographic areas are acquired to generate respective detection maps, the combination of the detection maps generated for one of the plurality of geographic zones comprising a normalisation on the basis of the number of images acquired for said geographic area.

11. Method according to one of the preceding claims, wherein the value assigned to a point i of the image is proportional to exp[-dᵢ²/(2σ²)], where dᵢ denotes the distance of the point i from the nearest hydrocarbon leak trace (2), and σ² represents a variance representative of an area of influence of the hydrocarbon leak trace.

12. Method according to one of the preceding claims, wherein the combination of the detection maps (9) generated comprises, for each point i, adding up the values assigned to point i on each detection map.
